# EUROPÄISCHE PATENTSCHRIFT

(11) **EP 3 856 007 B1**
(45) Veröffentlichungstag und Bekanntmachung des Hinweises auf die Patenterteilung: **02.11.2022**
(21) Anmeldenummer: 19778978.7
(22) Anmeldetag: 26.09.2019
(51) Int. Cl.: A61B 3/107

(54) **VERFAHREN ZUR BESTIMMUNG DER TOPOGRAPHIE DER KORNEA EINES AUGES**
METHOD FOR DETERMINING THE TOPOGRAPHY OF THE CORNEA OF AN EYE
PROCÉDÉ POUR LA DÉTERMINATION DE LA TOPOGRAPHIE DE LA CORNÉE D'UN OEIL

(30) Priorität: 28.09.2018 DE 102018216673
(43) Veröffentlichungstag der Anmeldung: 04.08.2021
(73) Patentinhaber: Carl Zeiss Meditec AG, 07745 Jena (DE)
(72) Erfinder: BAJRAMOVIC, Ferid, 94437 Mamming (DE); SCHÖBEL, Konrad, 99423 Weimar (DE); PLATT, Christian, 89073 Ulm (DE); CHEN, Wei-Jun, 07751 Jena (DE)
(74) Vertreter: Kintzel, Klaus-Peter
(86) Internationale Anmeldenummer: PCT/EP2019/075964
(87) Internationale Veröffentlichungsnummer: WO 2020/064895

(56) Entgegenhaltungen:
- EP-A1- 2 314 200
- DE-A1- 10 333 558
- DE-A1-102016 216 611

## Beschreibung

Die vorliegende Erfindung betrifft ein Verfahren zur Bestimmung der Topographie der Kornea eines Auges durch Auswertung des Spiegelbildes eines räumlich verteilten Musters, insbesondere eines in Ringen angeordneten Punktmusters.

Eine dreidimensionale Vermessung geometrischer Oberflächen, in unserem Fall der Hornhaut eines Auges, wird auch als Topografie bezeichnet. Die Oberflächenvermessung der Hornhaut des menschlichen Auges erweist sich dahingehend schwierig, dass die Messung unter anderem durch Augenbewegungen, Lidschlag, Wimpern, Tränenfilm und Verunreinigungen des Auges erheblich gestört wird.

Die Hornhaut (Kornea) ist der vorderste Abschnitt des Auges und weist eine vorgewölbte, spezifische Geometrie auf. Um diese geometrische Form der Hornhautvorderfläche in ihrer Gesamtheit zu erfassen bedient man sich der Topographie. Die Vorderflächenform kann dabei variieren, wobei zwei der wichtigsten und auch allgemein bekanntesten, die sphärische (kugelförmig) und die astigmatische (verkrümmte) Hornhaut sind.

Für die Erfassung der geometrischen Form der Hornhautvorderfläche wird aus einem auf die Vorderseite der Hornhaut mittels einer Projektionsoptik abgebildeten und mit Hilfe einer Kamera aufgenommenen Musters die Topographie der Hornhautoberfläche rekonstruiert. Entscheidend ist hierbei die Zuordnung der einzelnen Elemente des Musters zu den entsprechenden Elementen des (gleichen) Musters für eine entsprechende Referenzfläche. Aus den Abweichungen der Positionen der einzelnen Elemente des Musters lassen sich die Abweichungen der geometrischen Form der Hornhaut gegenüber dieser Referenzfläche ermitteln.

Nach dem bekannten Stand der Technik ist bei sogenannten Topographen die Verwendung von Placido-Scheiben zur Erzeugung konzentrischer Ringe am weitesten verbreitet. Weiterhin haben sich auch Topografen etabliert, die ein Punktmuster mit bis zu 10.000 Messpunkten auf die zu vermessende Hornhaut abbilden. Dies hat den Vorteil, dass sich die Hornhaut hochgenau vermessen lässt. Eine sehr hohe Anzahl an Messpunkten (z. B. > 1000) kann aber auch den Nachteil haben, dass mit steigender Dichte an Messpunkten die Komplexität und die Fehleranfälligkeit ihrer Zuordnung zum Referenzmuster steigt. Auch steigt die Gefahr, dass sich benachbarte Messpunkte berühren und in der weiteren Verarbeitung nicht trennen lassen (ähnlich sich berührender Ringe, siehe oben).

Die in der DE 10 2011 102 355 A1 beschriebene Lösung betrifft ein System zur Bestimmung der Oberflächenform der Kornea durch Auswertung des Spiegelbildes eines räumlich verteilten Ringmusters, wobei diese von einem gefresnelten Axicon mit ringförmigen Strukturen unterschiedlicher Radien erzeugt wird. Dazu wird das gefresnelte Axicon von einer Beleuchtungseinheit vollflächig beleuchtet. Die Auswertung des Spiegelbildes des räumlich verteilten Ringmusters erfolgt mittels einer telezentrischen, entfernungsunabhängigen Bilderfassung.

Nachteilig an dieser Lösung ist die Verwendung von reflektierenden Flächen im Material, da sich hier Oberflächenfehler wesentlich stärker auf die Kollimation des einfallenden Lichtes auswirken als bei brechenden Flächen. Weiterhin verlaufen die Strahlen zwischen Lichtquelle und der Kollimationsoptik divergent, so dass in diesem Bauraum geringere freie Durchmesser zur Verfügung stehen, als zwischen Kollimationsoptik und gefresneltem Axicon.

Weiterhin führt die Beleuchtung dazu, dass bei einer kurzen Brennweite große Einfallswinkel auftreten, die wiederum zu Reflexverlusten in den äußeren Zonen der Fresnelstruktur führen. Durch eine Verlängerung der Brennweite könnte dieses Problem zwar behoben werden, würde aber auch zu einer weiteren Verringerung des freien Bauraumes führen.

Die DE 10 2016 216611 A1 beschreibt ein entfernungsunabhängiges Messsystem, welches ein Punktmuster auf die zu vermessende Hornhaut abbildet. Das in der DE 10 2011 102 355 A1 beschriebene gefresnelte Axicon wurde dafür dahingehend verändert, dass die ringförmigen Strukturen über zusätzliche Facettenlinsen verfügen, wodurch vorteilhafte optische Eigenschaften der Projektion erreicht werden. Als weiteren Vorteil weist die hier beschriebene Lösung eine geringe Einbautiefe auf. Zudem lässt sich das gefresnelte Axicon preisgünstig und einfach als Spritzgussteil fertigen.

Das resultierende Muster besteht aus mehreren, konzentrisch angeordneten Ringen aus beispielsweise äquidistant angeordneten Punkten, wobei auch hier für die Beobachtungsoptik ein zentraler, innerer Bereich frei bleibt. Diese spezielle Anordnung der Punkte spielt für die nachfolgend beschriebene Erfindung aber keine Rolle.

In einem ersten Bildverarbeitungsschritt werden zunächst die einzelnen telezentrisch erfassten Punkte des innersten Ringes im Bild detektiert und anhand von Polarwinkeln jeder Punkt einem Referenzpunkt zugeordnet. Die restlichen Punkte werden dann iterativ zugeordnet, und zwar durch lokale Zuordnung von Punkten in der Umgebung bereits zugeordneter Punkte.

Nachteilig wirkt sich bei diesem Verfahren aus, dass es sehr sensibel gegenüber lokalen und globalen Störungen bzw. Deformationen des Punktmusters ist, wodurch sich ein lokaler Zuordnungsfehler über große Teile des Musters fortpflanzen kann.

Um dies zu vermeiden wird in der noch nicht veröffentlichten Anmeldung DE 10 2017 223512.8 eine Lösung beschrieben, die eine schnelle, einfache und sichere Zuordnung der einzelnen Elemente des Musters zu den entsprechenden Elementen des Musters für eine entsprechende Referenzfläche dadurch erlaubt, dass auf die Kornea ein Punktmuster in Form von zwei oder mehr Teilmustern nacheinander, d. h. in möglichst kurzem Zeitabstand abgebildet wird. Ein Fortpflanzen lokaler Zuordnungsfehler über große Teile des Musters lässt sich mit der beschriebenen Lösung zwar eingrenzen, aber nicht vermeiden, da viele der Störungen in den Punktbildern durch Augenbewegungen, Tränenfilmdynamik sowie Bewegungen des Augenlids und der Wimpern zeitlich veränderlich sind.

Die EP 2 314 200 A1 beschreibt den Gegenstand der Erfindung, der zum Stand der Technik gehört.

Der vorliegenden Erfindung liegt die Aufgabe zugrunde, eine Lösung zur Bestimmung der Topographie der Kornea eines Auges zu entwickeln, die die Nachteile der aus dem Stand der Technik bekannten Lösungen behebt und eine zuverlässige Rekonstruktion der Topographie der Hornhautoberfläche erlaubt. Dabei soll die Topographie der Hornhautoberfläche aus dem aufgenommenen Reflexbild eines auf die Vorderseite der Hornhaut projizierten Musters und der Zuordnung der einzelnen Elemente des Musters zu den entsprechenden Elementen eines Referenzmusters einer Referenzfläche rekonstruiert werden.

Erfindungsgemäß wird diese Aufgabe durch das vorgeschlagene Verfahren zur Bestimmung der Topographie der Kornea eines Auges, basierend auf der Detektion und Zuordnung eines von einer keratometrischen Messanordnung erzeugten Punktmusters in Bezug zu einem Referenzmuster und einer anschließenden mathematischen Ermittlung der Topographie, dadurch gelöst, dass bei der reflektiven Messung mehrere Reflexbilder nacheinander aufgenommen, dass in allen Reflexbildern alle Elemente detektiert und deren Koordinaten bestimmt werden, dass aus allen Reflexbildern ein Referenzbild ausgewählt wird, dass eine globale Verschiebung des Musters bezüglich des Referenzbildes ermittelt und kompensiert wird, dass für das Referenzbild eine Datenstruktur erzeugt und gespeichert wird, die die Koordinaten aller detektierten Elemente und einen Zähler mit dem Zählerstand 1 enthält, dass versucht wird die detektierten Elemente eines jeden Reflexbildes den Koordinaten des Referenzbildes zuzuordnen, dass für zugeordnete Elemente in der Datenstruktur des Referenzbildes der Zähler für das jeweilige Element erhöht wird, dass nicht zugeordnete Elemente aus den Reflexbildern als neue Koordinaten in der Datenstruktur mit Zählerstand 1 aufgenommen werden, dass die einem Element in der Datenstruktur zugeordneten Koordinaten geeignet gemittelt werden und dass für die Zuordnung zum Referenzmuster die aus allen Reflexbildern erzeugte Datenstruktur verwendet wird.

Bevorzugte Weiterbildungen und Ausgestaltungen sind Gegenstand der abhängigen Ansprüche.

Einer ersten vorteilhaften Ausgestaltung entsprechend werden die Reflexbilder bei der reflektiven Messung in Form einer Videosequenz aufgenommen.

Für die Detektion der Elemente in den Reflexbildern sowie für die Ermittlung der globalen Verschiebungen der Muster bezüglich eines aus der Videosequenz gewählten Referenzbildes werden bekannte Bildverarbeitungsmethoden verwendet.

Einer weiteren vorteilhaften Ausgestaltung entsprechend besteht das bei der keratometrischen Messanordnung erzeugte Muster aus Punkten.

Einer weiteren vorteilhaften Ausgestaltung entsprechend wird das Verfahren nicht im Nachverarbeitungs- sondern im Streaming- bzw. Echtzeit-Modus betrieben. Dies ist möglich, da die Topographie zu jedem Zeitpunkt aus der bis zu diesem Zeitpunkt vorliegenden Datenstruktur rekonstruiert werden kann, wobei die Qualität der Rekonstruktion mit der Anzahl der Aufnahmen steigt.

Obwohl die vorgeschlagene technische Lösung speziell für die Bestimmung der Oberflächenform, d. h. der Topografie, der Vorderfläche der Kornea eines Auges vorgesehen ist, kann sie prinzipiell auch für andere technische Gebiete eingesetzt werden, bei denen die Topografie einer geometrischen Oberfläche bestimmt werden soll oder bei denen variierende Punktmengen zeitlich verfolgt werden sollen, wie etwa in der Bildverarbeitung zum Tracken von Keypoints in Videosequenzen.

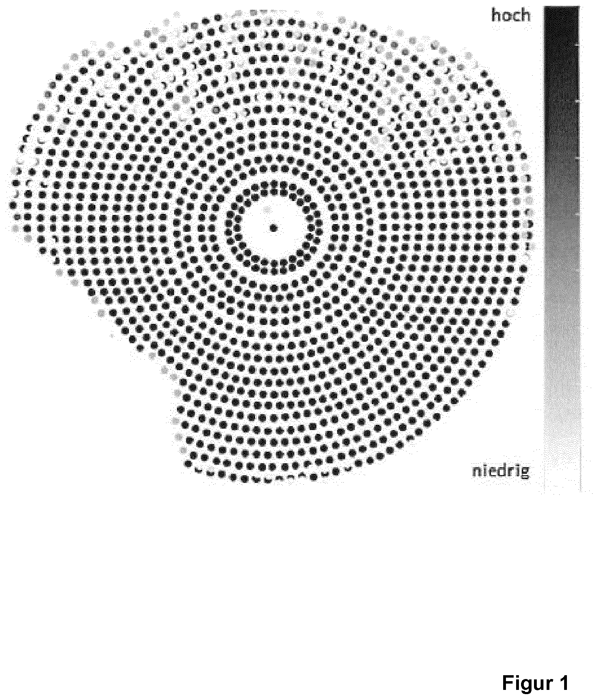

Die Erfindung wird nachfolgend anhand eines Ausführungsbeispiels näher beschrieben. Hierzu zeigt die
- Figur 1:: ein aus den Koordinaten und Zählerständen des Bezugsbildes resultierendes Punktmuster, bei dem die unterschiedlichen Grautöne der Lichtpunkte ihren jeweiligen Zählerstand repräsentieren.

Das vorgeschlagene Verfahren zur Bestimmung der Topographie der Kornea eines Auges basiert auf der Detektion und Zuordnung eines von einer keratometrischen Messanordnung erzeugten Musters in Bezug zu einem Referenzmuster und einer anschließenden mathematischen Ermittlung der Topographie.

Erfindungsgemäß werden dazu bei der reflektiven Messung mehrere Reflexbilder nacheinander aufgenommen, in allen Reflexbildern alle Elemente detektiert und deren Koordinaten bestimmt, aus allen Reflexbildern ein Bezugsbild ausgewählt, eine globale Verschiebung des Musters der Reflexbilder bezüglich des Bezugsbildes ermittelt und kompensiert und für das Bezugsbild eine Datenstruktur erzeugt und gespeichert, die die Koordinaten aller detektierten Elemente und einen Zähler mit dem Zählerstand 1 enthält.

Im Weiteren wird versucht die detektierten Elemente zumindest eines Teiles der Reflexbilder den Koordinaten des Bezugsbildes zuzuordnen, wobei für zugeordnete Elemente in der Datenstruktur des Bezugsbildes der Zähler für das jeweilige Element erhöht wird, nicht zugeordnete Elemente aus den Reflexbildern als neue Koordinaten in die Datenstruktur des Bezugsbildes mit Zählerstand 1 aufgenommen werden und die einem Element in der Datenstruktur zugeordneten Koordinaten geeignet gemittelt werden. Hierbei spielt die Reihenfolge der Verarbeitung der Reflexbilder keine Rolle. Für die Zuordnung zum Referenzmuster wird hierbei die aus den Reflexbildern erzeugte Datenstruktur des Bezugsbildes verwendet.

Als Bezugsbild kann einfach ein Reflexbild wie etwa das erste, fünfte oder auch das letzte der Sequenz ausgewählt werden, oder es wird mindestens ein Qualitätskriterium angewendet. Insbesondere kann das Bezugsbild im Verlaufe der Verarbeitung der Einzelbilder auch aktualisiert werden, falls das Qualitätskriterium für das aktuelle Bild besser ausfällt. Für eine Auswahl des Bezugsbildes bietet sich beispielsweise die maximale Anzahl der detektierten Elemente oder das zeitlich erste Reflexbild dessen Anzahl an detektierten Elementen einen vorgegebenen Wert übersteigt.

Hierzu werden mindestens 2, vorzugsweise 10 und besonders bevorzugt 100 oder mehr Reflexbilder der reflektiven Messung aufgenommen.

Vorzugsweise werden die Reflexbilder der reflektiven Messung in Form von Videosequenzen mit einer Aufnahmegeschwindigkeit von mindestens 1, vorzugsweise 5 oder mehr Reflexbildern pro Sekunde aufgenommen.

Da die durch Augenbewegungen, Tränenfilmdynamik sowie Bewegungen des Augenlids und der Wimpern hervorgerufenen Störungen zeitlich veränderlich sind, enthalten die zu unterschiedlichen Zeitpunkten aufgenommenen Reflexbilder dadurch im Allgemeinen verschiedene Störungen. Daher ist es vorteilhaft, mehr als ein Reflexbild aufzunehmen, wobei sich dabei Aufnahmen in Form von Videosequenzen anbieten.

Da sich Zuordnungsfehler in Einzelbildern schwer erkennen lassen, ist es günstig, die Bildsequenz zunächst ohne Bezug zum Referenzmuster zu verarbeiten. Stattdessen werden Elemente zwischen den einzelnen Reflexbildern der Sequenz zugeordnet. Dies erfolgt zumindest über einen Teil, vorzugsweise aber über die gesamte Sequenz.

Bei einer Aufnahme von Teilmustern nach Anmeldung DE 10 2017 223512.8 kann beim vorliegenden Verfahren darauf verzichtet werden, die Bilder in sehr kurzem zeitlichen Abstand aufzunehmen, und stattdessen eine Aufnahme der Teilmuster in beliebigem zeitlichen Abstand erfolgen, sofern sich die Teilmuster überlappen oder zusätzlich genügend Vollbilder aufgenommen werden. Dies vereinfacht die zeitliche Synchronisation von Beleuchtung und Kamera.

Da die zeitliche Varianz der Elemente des Musters wesentlich geringer ausfällt als die Abweichung vom Referenzmuster, lassen sich einander entsprechende Elemente zwischen verschiedenen Reflexbildern einer Sequenz wesentlich einfacher zuordnen, als zwischen einem einzelnen Reflexbild und dem Referenzmuster.

Um die Elemente in den Reflexbildern zu detektieren und deren Koordinaten zu bestimmen werden bekannte Bildverarbeitungsmethoden verwendet.

Auch für die Ermittlung und Kompensation der globalen Verschiebung des Musters bezüglich des Bezugsbildes werden bekannte Bildverarbeitungsmethoden angewendet, wobei die Kreuzkorrelation oder eine Variante davon zu bevorzugen ist.

Die Ermittlung und Kompensation der globalen Verschiebung des Musters bezüglich des Bezugsbildes erfolgt hierbei ohne konkreten Bezug zu den einzelnen Elementen.

Durch diesen Schritt können Augenbewegungen kompensiert werden, da sich kleine Rotationen großenteils durch Translation des Musters in den Reflexbildern darstellen.

Für die Zuordnung eines detektierten Elementes eines Reflexbildes zu den entsprechenden Koordinaten des Bezugsbildes wird erfindungsgemäß ein Schwellwert bezüglich deren Abstandes verwendet. Als Schwellwert wird beispielsweise der mittlere Durchmesser der Elemente vorgegeben.

Für jedes Reflexbild wird zunächst versucht, die detektierten Elemente mit ihren Koordinaten den betreffenden Elementen des Bezugsbildes zuzuordnen. Dazu wird für jedes Element des (verschiebungskompensierten) Reflexbildes dessen nächster Nachbar im Bezugsbild bestimmt. Ist dessen nächster Nachbar im Reflexbild das ursprüngliche Element und liegt der Abstand beider Elemente unter dem vorgegebenen Schwellwert, so wird das detektierte Element zugeordnet, andernfalls nicht. Dabei wird der Zählerstand des betreffenden Elementes des Bezugsbildes um den Wert 1 erhöht und seine Koordinaten werden geeignet mit denen des detektierten Elementes gemittelt.

Vorzugsweise werden vor dem Mitteln der für ein Element in der Datenstruktur des Bezugsbildes zugeordneten Koordinaten sogenannte Ausreißer entfernt.

Detektierte Elemente, die nicht zugeordnet werden konnten, werden am Ende als neue Elemente mit ihren Koordinaten und einem Zählerstand von 1 zur Datenstruktur hinzugefügt. Mit allen weiteren Reflexbildern wird im Anschluss ebenso verfahren.

Erfindungsgemäß erfolgt die Zuordnung der erzeugten Datenstruktur zum Referenzmuster in Abhängigkeit vom Wert der Zählerstände der Elemente vorzugsweise mehrstufig, wobei mit den höchsten Zählerständen begonnen wird.

Für die Zuordnung zum Referenzmuster werden nicht die Elemente der einzelnen Reflexbilder, sondern die erzeugte Datenstruktur des Bezugsbildes verwendet.

Diese Datenstruktur enthält Sequenzen detektierter Elemente, die in ein oder mehreren einzelnen Reflexbildern einander zugeordnet wurden. Dabei entsprechen die Werte der Zählerstände der Anzahl der Reflexbilder in denen die entsprechenden Elemente detektiert wurden. Die Elemente mit den größten Zählerständen weisen demnach die höchste Verlässlichkeit auf.

Vorzugsweise können die Elemente mit den größten Zählerständen zusätzlich als Wichtung beim Anfitten einer Freiformfläche an die Koordinaten verwendet werden.

Einer bevorzugten Ausgestaltung entsprechend wird von der keratometrischen Messanordnung ein Muster aus Punkten erzeugt. Ein derartiges Punktmuster kann beispielsweise mit Hilfe einer Facettenlinse erzeugt werden, wobei das erzeugte Punktmuster aus mehreren, beispielsweise konzentrischen Ringen mit vorzugsweise äquidistant angeordneten Punkten besteht.

Dadurch wird es ermöglicht, die Koordinaten und Zählerstände aller in der Datenstruktur des Bezugsbildes gespeicherten Elemente als projiziertes Muster aus Lichtpunkten mit unterschiedlichen, den Zählerständen entsprechenden Graustufen darzustellen.

Hierzu zeigt die **Figur 1** ein aus den Koordinaten und Zählerständen des Bezugsbildes resultierendes Punktmuster. Hierbei entsprechend die unterschiedlichen Grautöne der Lichtpunkte ihrem jeweiligen Zählerstand, wobei dunklere Punkte einen höheren Zählerstand repräsentieren.

Vorzugsweise lässt sich das Muster aus Lichtpunkten der mathematisch ermittelten Topographie der Kornea überlagert dargestellten.

Es ist aber auch denkbar, dass die lokale Verlässlichkeit der Topographie lediglich durch die Zählerstände bewertet und dies in der Topographiekarte visualisiert wird. Auch hierfür würde sich eine Überlagerung mit hellen oder dunklen Punkten, unabhängig vom projizierten Punktmustern anbieten.

Mit der vorliegenden Erfindung wird eine Lösung zur Bestimmung der Topographie der Kornea eines Auges zur Verfügung gestellt, die die Nachteile der aus dem Stand der Technik bekannten Lösungen behebt und eine zuverlässige Rekonstruktion der Topographie der Hornhautoberfläche erlaubt. Dabei wird die Topographie der Hornhautoberfläche aus dem aufgenommenen Reflexbild eines auf die Vorderseite der Hornhaut projizierten Musters und Zuordnung der einzelnen Elemente des Musters zu den entsprechenden Elementen eines Referenzmusters einer Referenzfläche rekonstruiert.

Obwohl die vorgeschlagene technische Lösung speziell für die Bestimmung der Oberflächenform, d. h. der Topografie, der Vorderfläche der Kornea eines Auges vorgesehen ist, kann sie prinzipiell auch für andere technische Gebiete eingesetzt werden, bei denen die Topografie einer geometrischen Oberfläche bestimmt werden soll oder bei denen variierende Punktmengen zeitlich verfolgt werden sollen, wie etwa in der Bildverarbeitung zum Tracken von Keypoints in Videosequenzen.

Im Unterschied zu den aus dem Stand der Technik bekannten Lösungen werden bei dem hier vorgeschlagenen Verfahren zum einen Bildsequenz statt Einzelbild aufgenommen und zum anderen auch nicht Einzelbilder einem Referenzmuster zugeordnet sondern einer aus den Einzelbildern der Bildsequenz resultierenden Datenstruktur.

## Patentansprüche

1. Verfahren zur Bestimmung der Topographie der Kornea eines Auges, basierend auf der Detektion und Zuordnung eines von einer keratometrischen Messanordnung erzeugten Musters in Bezug zu einem Referenzmuster und auf einer anschließenden mathematischen Ermittlung der Topographie, wobei
bei einer reflektiven Messung mehrere Reflexbilder nacheinander aufgenommen, in allen Reflexbildern alle Elemente des erzeugten Musters detektiert und deren Koordinaten bestimmt werden, **dadurch gekennzeichnet**
**dass** aus allen Reflexbildern ein Bezugsbild ausgewählt wird, dass eine globale Verschiebung des Musters aller Reflexbilder bezüglich des Bezugsbildes ermittelt und kompensiert wird, dass für das Bezugsbild eine Datenstruktur erzeugt und gespeichert wird, die die Koordinaten aller detektierten Elemente und einen Zähler mit dem Zählerstand 1 enthält, dass versucht wird die detektierten Elemente zumindest eines Teiles der Reflexbilder den den detektierten Elementen entsprechenden Koordinaten des Bezugsbildes zuzuordnen, dass für Elemente, die zugeordnet werden konnten ,
in der Datenstruktur des Bezugsbildes deren Zähler um 1 erhöht wird, dass nicht zugeordnete Elemente aus den Reflexbildern als neue Koordinaten in der Datenstruktur des Bezugsbildes mit Zählerstand 1 aufgenommen werden, dass die einem Element in der Datenstruktur des Bezugsbildes zugeordneten Koordinaten geeignet gemittelt werden und dass für die Zuordnung zum Referenzmuster die aus allen Reflexbildern erzeugte Datenstruktur des Bezugsbildes verwendet wird.

2. Verfahren nach Anspruch 1, **dadurch gekennzeichnet, dass** mindestens 2, vorzugsweise 10 und besonders bevorzugt 100 oder mehr Reflexbilder der reflektiven Messung aufgenommen werden.

3. Verfahren nach den Ansprüchen 1 und 2, **dadurch gekennzeichnet, dass** die Reflexbilder der reflektiven Messung in Form von Videosequenzen mit einer Aufnahmegeschwindigkeit von mindestens 1, vorzugsweise 5 oder mehr Reflexbildern pro Sekunde aufgenommen werden.

4. Verfahren nach Anspruch 1, **dadurch gekennzeichnet, dass** Bildverarbeitungsmethoden verwendet werden, um die Elemente in den Reflexbildern zu detektieren und deren Koordinaten zu bestimmen.

5. Verfahren nach Anspruch 1, **dadurch gekennzeichnet, dass** für die Auswahl des Bezugsbildes mindestens ein Qualitätskriterium Verwendung findet.

6. Verfahren nach den Ansprüchen 1 und 5, **dadurch gekennzeichnet, dass** als Qualitätskriterium die Anzahl der detektierten Elemente verwendet wird.

7. Verfahren nach Anspruch 1, **dadurch gekennzeichnet, dass** die globale Verschiebung des Musters bezüglich des Bezugsbildes mit Hilfe von Bildverarbeitungsmethoden ermittelt und kompensiert wird.

8. Verfahren nach den Ansprüchen 1 und 7, **dadurch gekennzeichnet, dass** als Bildverarbeitungsmethode die Kreuzkorrelation oder eine Variante davon verwendet wird.

9. Verfahren nach Anspruch 1, **dadurch gekennzeichnet, dass** für die Zuordnung eines detektierten Elementes eines jeden Reflexbildes zu den entsprechenden Koordinaten des Bezugsbildes ein Schwellwert bezüglich deren Abstandes vorgegeben wird.

10. Verfahren nach den Ansprüchen 1 und 9, **dadurch gekennzeichnet, dass** als Schwellwert bezüglich des Abstandes der mittlere Durchmesser der Elemente vorgegeben wird.

11. Verfahren nach Anspruch 1 und 10, **dadurch gekennzeichnet, dass** für die Zuordnung eines detektierten Elementes eines jeden Reflexbildes zu den entsprechenden Koordinaten des Bezugsbildes Nächste-Nachbarn-Beziehungen verwendet werden.

12. Verfahren nach Anspruch 1, **dadurch gekennzeichnet, dass** vordem Mitteln der für ein Element in der Datenstruktur des Bezugsbildes zugeordneten Koordinaten sogenannte Ausreißer entfernt werden.

13. Verfahren nach Anspruch 1, **dadurch gekennzeichnet, dass** die Zuordnung der erzeugten Datenstruktur des Bezugsbildes zum Referenzmuster in Abhängigkeit vom Zählerstand der Elemente mehrstufig erfolgt, wobei mit den größten Zählerständen begonnen wird.

14. Verfahren nach den Ansprüchen 1 und 13, **dadurch gekennzeichnet, dass** besonders kleine Zählerstände von der Zuordnung ganz ausgeschlossen werden.

15. Verfahren nach Anspruch 1, **dadurch gekennzeichnet, dass** es im Streaming- bzw. Echtzeit-Modus betrieben wird.

16. Verfahren nach Anspruch 1, **dadurch gekennzeichnet, dass** das Bezugsbild aktualisiert wird.

17. Verfahren nach den Ansprüchen 1 und 2, **dadurch gekennzeichnet, dass** der Wert der Zählerstände ein Maß für die Verlässlichkeit der Zuordnung der Elemente darstellt und als Gewichtung beim Anfitten einer Freiformfläche an die Koordinaten verwendet wird.

18. Verfahren nach Anspruch 1, **dadurch gekennzeichnet, dass** das bei der keratometrischen Messanordnung erzeugte Muster aus Punkten besteht.

19. Verfahren nach Anspruch 1, **dadurch gekennzeichnet, dass** die Koordinaten und Zählerstände aller in der Datenstruktur gespeicherten Elemente als projiziertes Muster aus Lichtpunkten mit unterschiedlichen, den Zählerständen entsprechenden Graustufen dargestellt sind.

20. Verfahren nach Anspruch 1, **dadurch gekennzeichnet, dass** das dargestellte Muster aus Lichtpunkten der mathematisch ermittelten Topographie der Kornea überlagert wird.

## Claims

1. Method for determining the topography of the cornea of the eye, based on the detection and assignment of a pattern, produced by a keratometric measurement arrangement, with respect to a reference pattern and on a subsequent mathematical ascertainment of the topography, wherein in a reflective measurement a plurality of reflection images are recorded successively, all the elements of the pattern produced are detected in all the reflection images and the coordinates of said elements are determined, **characterized in that** a reference image is selected from all the reflection images, **in that** a global displacement of the pattern of all the reflection images with respect to the reference image is ascertained and compensated, **in that** a data structure containing the coordinates of all the detected elements and a counter with the counter reading 1 is produced and stored for the reference image, **in that** an attempt is made to assign the detected elements of at least some of the reflection images to the coordinates of the reference image that correspond to the detected elements, **in that**, for elements that could be assigned, the counter of the data structure of the reference image is increased by 1 in said data structure, **in that** elements from the reflection images that were not assigned are included as new coordinates in the data structure of the reference image with a counter reading 1, **in that** the coordinates assigned to an element in the data structure of the reference image are appropriately averaged, and **in that** the data structure of the reference image produced from all the reflection images is used for the assignment to the reference pattern.

2. Method according to Claim 1, **characterized in that** at least 2, preferably 10 and with particular preference 100 or more reflection images of the reflective measurement are recorded.

3. Method according to Claims 1 and 2, **characterized in that** the reflection images of the reflective measurement are recorded in the form of video sequences with a recording speed of at least 1, preferably 5 or more reflection images per second.

4. Method according to Claim 1, **characterized in that** image processing methods are used to detect the elements in the reflection images and to determine their coordinates.

5. Method according to Claim 1, **characterized in that** at least one quality criterion is used for the selection of the reference image.

6. Method according to Claims 1 and 5, **characterized in that** the quality criterion used is the number of the detected elements.

7. Method according to Claim 1, **characterized in that** the global displacement of the pattern with respect to the reference image is ascertained and compensated with the aid of image processing methods.

8. Method according to Claims 1 and 7, **characterized in that** the cross correlation or a variant thereof is used as the image processing method.

9. Method according to Claim 1, **characterized in that** for the assignment of a detected element of each reflection image to the corresponding coordinates of the reference image a threshold value with respect to the distance between them is specified.

10. Method according to Claims 1 and 9, **characterized in that** the specified threshold value with respect to the distance is the average diameter of the elements.

11. Method according to Claim 1 and 10, **characterized in that** nearest-neighbour relationships are used for the assignment of a detected element of each reflection image to the corresponding coordinates of the reference image.

12. Method according to Claim 1, **characterized in that** what are known as outliers are removed before the coordinates assigned for an element in the data structure of the reference image are averaged.

13. Method according to Claim 1, **characterized in that** the assignment of the produced data structure of the reference image to the reference pattern takes place in multiple stages depending on the counter reading of the elements, wherein the greatest counter readings are dealt with first.

14. Method according to Claims 1 and 13, **characterized in that** particularly small counter readings are entirely excluded from the assignment.

15. Method according to Claim 1, **characterized in that** it is carried out in streaming or real-time mode.

16. Method according to Claim 1, **characterized in that** the reference image is updated.

17. Method according to Claims 1 and 2, **characterized in that** the value of the counter readings represents a measure of the reliability of the assignment of the elements and is used as weighting when fitting a free-form surface to the coordinates.

18. Method according to Claim 1, **characterized in that** the pattern produced in the keratometric measurement arrangement consists of dots.

19. Method according to Claim 1, **characterized in that** the coordinates and counter readings of all the elements stored in the data structure are presented as a projected pattern of light dots having different greyscales corresponding to the counter readings.

20. Method according to Claim 1, **characterized in that** the presented pattern of light dots is overlaid on the mathematically ascertained topography of the cornea.

## Revendications

1. Procédé de détermination de la topographie de la cornée d'un œil, sur la base de la détection et de l'association d'un motif, généré par un dispositif de mesure appelé kératomètre, par rapport à un motif de référence et d'une détermination mathématique ultérieure de la topographie,
lors d'une mesure par réflexion plusieurs images réfléchies étant enregistrées les unes après les autres, tous les éléments du motif généré étant détectés et leurs coordonnées déterminées dans toutes les images réfléchies,
**caractérisé en ce que**
une image de référence est sélectionnée parmi toutes les images réfléchies,
un décalage global du motif de toutes les images réfléchies par rapport à l'image de référence est déterminé et compensé,
une structure de données est générée et mémorisée pour l'image de référence, laquelle structure de données contient les coordonnées de tous les éléments détectés et un compteur à la position 1,
une tentative est effectuée pour associer les éléments détectés d'au moins une partie des images réfléchies aux coordonnées de l'image de référence qui correspondent aux éléments détectés,
en ce qui concerne les éléments de la structure de données de l'image de référence qui ont pu être associés, leur compteur est incrémenté de 1,
les éléments des images réfléchies qui n'ont pas été associés sont inclus comme nouvelles coordonnées dans la structure de données de l'image de référence avec une position de compteur à 1,
les coordonnées associées à un élément de la structure de données de l'image de référence sont moyennées de manière appropriée et
la structure de données de l'image de référence qui a été générée à partir de toutes les images réfléchies est utilisée pour l'association au motif de référence.

2. Procédé selon la revendication 1, **caractérisé en ce qu'**au moins 2, de préférence 10 et de manière particulièrement préférée 100, images réfléchies ou plus de la mesure de réflexion sont enregistrées.

3. Procédé selon les revendications 1 et 2, **caractérisé en ce que** les images réfléchies de la mesure de réflexion sont enregistrées sous forme de séquences vidéo à une vitesse d'enregistrement d'au moins 1, de préférence 5, images réfléchies ou plus par seconde.

4. Procédé selon la revendication 1, **caractérisé en ce que** des procédés de traitement d'images sont utilisés pour détecter les éléments dans les images réfléchies et déterminer leurs coordonnées.

5. Procédé selon la revendication 1, **caractérisé en ce qu'**au moins un critère de qualité est utilisé pour sélectionner l'image de référence.

6. Procédé selon les revendications 1 et 5, **caractérisé en ce que** le nombre d'éléments détectés est utilisé comme critère de qualité.

7. Procédé selon l'une des revendications précédentes, **caractérisé en ce que** le décalage global du motif par rapport à l'image de référence est déterminé et compensé à l'aide de méthodes de traitement d'images.

8. Procédé selon les revendications 1 et 7, **caractérisé en ce que** la corrélation croisée ou une variante de celle-ci est utilisée comme méthode de traitement d'images.

9. Procédé selon la revendication 1, **caractérisé en ce que**, pour associer un élément détecté de chaque image réfléchie aux coordonnées correspondantes de l'image de référence, une valeur seuil par rapport à leur distance est spécifiée.

10. Procédé selon les revendications 1 et 9, **caractérisé en ce que** le diamètre moyen des éléments est spécifié comme une valeur seuil par rapport à la distance.

11. Procédé selon les revendications 1 et 10, **caractérisé en ce que** les relations les plus proches sont utilisées pour associer un élément détecté de chaque image réfléchie aux coordonnées correspondantes de l'image de référence.

12. Procédé selon la revendication 1, **caractérisé en ce que** les valeurs dites aberrantes sont éliminées avant de moyenner les coordonnées associées à un élément de la structure de données de l'image de référence.

13. Procédé selon la revendication 1, **caractérisé en ce que** l'association de la structure de données générée de l'image de référence au motif de référence est effectuée en plusieurs étapes selon le nombre d'éléments, en commençant par les positions de compteur les plus élevées.

14. Procédé selon les revendications 1 et 13, **caractérisé en ce que** des positions de compteur notamment petites sont complètement exclues de l'association.

15. Procédé selon la revendication 1, **caractérisé en ce qu'**il est mis en œuvre en mode streaming ou temps réel.

16. Procédé selon la revendication 1, **caractérisé en ce que** l'image de référence est actualisée.

17. Procédé selon les revendications 1 et 2, **caractérisé en ce que** la valeur des positions de compteur représente une mesure de la fiabilité de l'association des éléments et est utilisée comme pondération lors de l'ajustement d'une surface de forme libre aux coordonnées.

18. Procédé selon la revendication 1, **caractérisé en ce que** le motif généré par le dispositif de mesure appelé kératomètre comprend des points.

19. Procédé selon la revendication 1, **caractérisé en ce que** les coordonnées et les positions de compteur de tous les éléments mémorisées dans la structure de données sont représentés sous la forme d'un motif projeté de points lumineux ayant différents niveaux de gris qui correspondent aux positions de compteur.

20. Procédé selon la revendication 1, **caractérisé en ce que** le motif représenté de points lumineux est superposé à la topographie de la cornée déterminée mathématiquement.
